# EUROPEAN PATENT APPLICATION

(11) **EP 2 088 207 A1**
(43) Date of publication of application: **12.08.2009**
(21) Application number: 09100120.6
(22) Date of filing: 28.11.2006
(51) Int. Cl.: C12Q 1/68, C12N 5/10, A01K 67/027, A01K 67/033

(54) **Biomarkers and methods for identification of agents useful in the treatment of affective disorders**

(30) Priority: 28.11.2005 GB 0524110
(62) Divisional of application: 06808694.1
(71) Applicant: Psynova Neurotech Limited, Cambridge CB4 OWS (GB)
(72) Inventor: Lockstone, Helen Elizabeth, Cambridge, CB2 1QT (GB); Wayland, Matthew Thomas, Cambridge, Cambridgeshire CB2 1QT (GB); Bahn, Sabine, Cambridge, Cambridgeshire CB2 1QT (GB); Ryan, Margaret, 9022 Dunedin (NZ)
(74) Representative: Perry, Robert Edward

(57) **Abstract**

A method of identifying a potential therapeutic agent for the prevention, treatment or amelioration of an affective disorder, comprises:
(a) contacting a cell with a candidate therapeutic agent *in vitro*;
(b) determining whether expression of the gene PREPL is modulated in the cell in response to the candidate therapeutic agent; and
(c) identifying the candidate as a potential therapeutic agent if expression of the gene is increased.

Methods of diagnosing and monitoring an affective disorder, comprising assessing the level of expression of the gene PREPL in a biological sample taken from a subject are also provided.

## Description

### Field of the Invention

The invention relates to methods of identifying agents useful in the treatment of affective disorders, such as bipolar disorder (BD), depression and anxiety disorders, and to methods of diagnosing affective disorders. The invention also relates to biomarkers for affective disorders and their use in methods of screening potential therapeutic agents, in diagnosis, in assessment of prognosis, in selection of candidate subjects for clinical trials and in monitoring of response to therapeutic intervention.

### Background of the Invention

Bipolar affective disorder (BD) is a severe remitting-relapsing psychiatric disorder with an estimated lifetime risk of about 1%. The disorder is characterised by alternating episodes of mania and depression and frequently presents with psychotic features indistinguishable from those seen in schizophrenia. The cost of BD is immense, in both financial and human terms. There is a strong hereditary component to BD, but environmental factors also seem to play a critical role in precipitating the disorder. Numerous linkage and association studies have been conducted in an attempt to identify specific genes and/or chromosomal regions that are involved in the pathophysiology of BD or confer susceptibility respectively. However, no specific gene has yet been definitively linked to the disorder, although studies have implicated chromosome regions such as 18p, 22q12 and Xq28¹⁻². Despite extensive research efforts, the underlying pathophysiology of BD remains elusive.

The ICD-10 Classification of Mental and Behavioural Disorders, published by the World Health Organization in 1992, is the manual most commonly used by European psychiatrists to diagnose mental health conditions. The Diagnostic and Statistical Manual of Mental Disorders fourth edition (DSM IV) published by the American Psychiatric Association, Washington D.C., 1994, describes the diagnostic criteria, subtypes, associated features and criteria for differential diagnosis of mental health disorders.

Two sub-types of bipolar illness have been defined sufficiently clearly to be given their own DSM categories: Bipolar I and Bipolar II. Bipolar I is a disorder characterized by manic episodes; the 'high' of the manic-depressive cycle. Generally this manic period is followed by a period of depression, although some bipolar I individuals may not experience a major depressive episode. Mixed states, where both manic or hypomanic symptoms and depressive symptoms occur at the same time, also occur frequently with bipolar I patients (for example, depression with the racing thoughts of mania). Also, dysphoric mania is common, this is mania characterized by anger and irritability.

Bipolar II: This disorder is characterized by major depressive episodes alternating with episodes of hypomania, a milder form of mania. Hypomanic episodes can be a less disruptive form of mania and may be characterized by low-level, non-psychotic symptoms of mania, such as increased energy or a more elated mood than usual. It may not affect an individual's ability to function on a day to day basis. The criteria for hypomania differ from those for mania only by their shorter duration (at least 4 days instead of 1 week) and milder severity (no marked impairment of functioning, hospitalization or psychotic features).

If alternating episodes of depressive and manic symptoms last for two years and do not meet the criteria for a major depressive or a manic episode then the diagnosis is classified as a Cyclothymic disorder, which is a less severe form of bipolar affective disorder. Cyclothymic disorder is diagnosed over the course of two years and is characterized by frequent short periods of hypomania and depressive symptoms separated by periods of stability.

Rapid cycling occurs when an individual's mood fluctuates from depression to hypomania or mania in rapid succession with little or no periods of stability in between. One is said to experience rapid cycling when one has had four or more episodes, in a given year, that meet criteria for major depressive, manic, mixed or hypomanic episodes. Some people who rapid cycle can experience monthly, weekly or even daily shifts in polarity (sometimes called ultra rapid cycling).

Diagnosis of Bipolar III has been used to categorise manic episodes which occur as a result of taking an antidepressant medication, rather than occurring spontaneously. Confusingly, it has also been used in instances where an individual experiences hypomania or cyclothymia (i.e. less severe mania) without major depression.

The DSM IV states that there are a number of criteria by which major depression is clinically defined. The condition must have been evident for at least two weeks and must have five of the following symptoms: a depressed mood for most of the day, almost every day, a loss of interest or pleasure in almost all activities, almost every day, changes in weight and appetite, sleep disturbance, a decrease in physical activity, fatigue and loss of energy, feelings of worthlessness or excessive feelings of guilt, poor concentration levels, suicidal thoughts.

Both the depressed mood and a loss of interest in everyday activities must be evident as two of the five symptoms which characterize a major depression. It is difficult to distinguish the symptoms of an individual suffering from the depressed mood of manic depression from someone suffering from a major depression. Dysthymia is a less severe depression than unipolar depression, but it can be more persistent.

Anxiety disorders as classified under the DSMIV include a wide range of disorders, including agoraphobia, panic disorder to obsessive compulsive disorder, post-traumatic stress disorder, and generalised anxiety disorder. In the ICD-10 these disorders are classified as "Neurotic, stress-related and somatoform disorders".

Gene expression profiling using microarray technology has become a promising approach for the investigation of complex disorders, as the expression levels of thousands of genes can be assessed simultaneously. Expression-profiling studies of post-mortem brain tissue derived from patients who suffered from psychiatric disorders have already been published, with the majority reporting changes in the schizophrenia brain³⁻⁷. Others have investigated both schizophrenia and bipolar disorder but few studies so far have focused on BD⁸⁻¹², depression or anxiety disorders.

A microarray analysis of BD using post-mortem brain samples from prefrontal cortex reported up-regulation of genes coding for stress response proteins and chaperones while receptor, channel and transporter genes were mainly down-regulated⁹.

A number of oligodendrocyte-related and myelination genes have been found to be altered in BD and schizophrenia¹¹. Nine such genes were identified as altered in BD using QPCR but only two were confirmed by microarray (FDR corrections were not applied in this study). This previous study showed the likely existence of subgroups with regard to myelination changes, *i*.*e.* some patients showed highly significant transcript down-regulations, whilst a small number of patients showed significant increases. Thus, the existence of subgroups, as well as alternative splice variants, may make it difficult to detect myelination changes using microarray technology.

Changes to mitochondria-related genes have been observed in both the DLPFC⁸ and the hippocampus¹⁰ of BD patients; in contrast to an earlier schizophrenia study³, here no significant evidence was found for mitochondrial dysfunction in the bipolar brain. However, down-regulation of ubiquitin pathway genes has been previously observed in bipolar disorder, in alcohol abuse and by several groups in schizophrenia patients, suggesting a common disease pathway. A recent functional genomics approach to identify candidate genes and pathways for BD found some changes, including signal transduction, neurotransmitter and synaptic genes⁴⁸.

The aetiology of affective disorders is poorly understood. There is a need for identification of markers and targets that can be used in screening and development of drugs for treating affective disorders such as bipolar disorder, depression and anxiety disorders. Such markers are also needed to improve methods for diagnosis and monitoring of affective disorders and to assess response to therapeutic intervention.

### Disclosure of Invention

The invention provides a method of identifying a potential therapeutic agent for the prevention, treatment, or amelioration of an affective disorder, which comprises:
(a) contacting a cell or cells with a candidate therapeutic agent, or
(b) administering a candidate therapeutic agent to an organism or organisms;
(c) determining if expression of one or more of the following genes is modulated in the cell(s) or organism(s) in response to the candidate therapeutic agent: AVPR2, AZU1, CELSR1, DRD2, FZD2, GCG, GPR132, GPR4, HTR7, IL8RB, MS4A2, OPN1 MW /// OPN1LW, OPRL1, OPRM1, OR3A2, OXTR, PARD3, RGS16, SSTR5, SSX2, VIPR2, FZD3, AZU1, C8A, CD7, CD72, CRIP1, GBX2, HLA-E, IL1R2, IL2RA, IL4, IL4R, IL5, IL8RB, KIR2DS1, KNG1, MS4A2, ORM1///ORM2, PFC, PRLR, TNFRSF17, SOCS5, WWP1, APG12L, FLJ11011, HIP2, PCGF4, PJA2, SENP6, TRIM23, UBE2G1, USP14, VPS41, WWP1, FBXL8, FLJ20315, BCAP29, C14orf108, COPA, FUSIP1, G3BP2, GRP58, HSPCA, IPO7, KIAA1012, KPNB1, RAB2, RANBP2, RBM8A, STAM, VPS41, STX11, TIMM17A, SYT1, CREB1, PREPL, SLC29A1, MYT1; and,
(d) identifying the candidate as a potential therapeutic agent if expression of one or more of the genes is modulated.

Affective disorders include bipolar disorders, depression and anxiety disorders.

Modulation of expression of one or more of the genes can be assessed by comparing the expression level of the gene or genes in the presence and absence of the candidate therapeutic agent. The term "modulated" is used herein to mean an upregulation, or downregulation, of expression of a gene. The term "modulator" is used herein to mean an upregulator, or downregulator, of expression of a gene.

The candidate is identified as a potential therapeutic agent if expression of one or more of the following genes is increased:

FZD3, SOCS5, WWP1, APG12L, FLJ11011, HIP2, PCGF4, PJA2, SENP6, TRIM23, UBE2G1, USP14, VPS41, WWP1, BCAP29, C14orf108, COPA, FUSIP1, G3BP2, GRP58, HSPCA, IPO7, KIAA1012, KPNB1, RAB2, RANBP2, RBM8A, STAM, VPS41, SYT1, CREB1, PREPL and/or SLC29A1; and/or,

if expression of one or more of the following genes is decreased:
AVPR2, AZU1, CELSR1, DRD2, FZD2, GCG, GPR132, GPR4, HTR7, IL8RB, MS4A2, OPN1MW /// OPN1LW, OPRL1, OPRM1, OR3A2, OXTR, PARD3, RGS16, SSTR5, SSX2, VIPR2, AZU1, C8A, CD7, CD72, CRIP1, GBX2, HLA-E, IL1R2, IL2RA, IL4, IL4R, IL5, IL8RB, KIR2DS1, KNG1, MS4A2, ORM1///ORM2, PFC, PRLR, TNFRSF17, FBXL8, FLJ20315; STX11, TIMM17A and/or MYT1.

The candidate can be identified as a potential therapeutic agent if expression of the majority of the genes is modulated in response to exposure to the candidate therapeutic agent. The term "majority" used herein means more than 50%, preferably at least 60%, more preferably at least 70%, more preferably at least 80%, more preferably at least 90%, yet more preferably at least 95%, most preferably all of the genes.

The cell or cells can be selected from the group consisting of: neuronal cells, glial cells or other brain cells, or peripheral cells, such as T-cells.

The organism can be a non-human organism, such as a non-human animal or an animal model for an affective disorder. Animal models include those in which affective disorders are simulated, e.g. by administration of a stimulant, such as an amphetamine to the animal. Any suitable animal may be used as a subject non-human animal, for example a non-human primate, horse, cow, pig, goat, sheep, dog, cat, fish (e.g. Zebra fish), rodent, e.g. guinea pig, rat or mouse; insect (e.g. *Drosophila*), amphibian (e.g. *Xenopus*) or *C. elegans.*

Methods and assays of the invention may be implemented using any method suitable for measuring changes in gene expression. Methods of determining the expression level of a gene are well known in the art. Modulation of expression can be identified by assessing the amount or concentration of mRNA, a nucleic acid derived from mRNA or a protein translated from the mRNA.

Gene expression may be measured by assessing mRNA levels using a method including reverse transcription and polymerase chain reactions ("RT-PCR"), such as quantitative PCR (in particular, real-time quantitative PCR), and Northern blotting. In one suitable method for determining the level of mRNA expressed, a total RNA sample is obtained from the cell(s) or organism(s), cDNA is synthesized from mRNA of the gene or genes of interest, and the cDNA is used for real-time quantitative PCR analysis to determine the level of the mRNA of interest in the sample. Systems and kits for implementing such methods are commercially available.

Examples of suitable methods for determining the level of protein expression are immunological methods, involving an antibody, or an antibody fragment capable of specific binding to the protein of interest. Suitable immunological methods include sandwich immunoassays, such as sandwich ELISA in which detection of the peptide is performed using two antibodies which recognize different epitopes; radioimmunoassays (RIA), direct or competitive enzyme linked immunosorbent assays (ELISA), enzyme immuno assays (EIA), western blotting, immunoprecipitation and any particle-based immunoassay (e.g. using gold, silver, or latex particles, magnetic particles or Q-dots). Immunological methods may be performed, for example, in microtitre plate or strip format.

A method for identifying a potential therapeutic agent according to the invention can be performed in an array format, this is particularly preferred in embodiments in which changes in expression level of a plurality of genes in response to the candidate therapeutic agent are assessed.

In a preferred embodiment, it is determined whether expression of the gene or genes is altered in the orbitofrontal cortex (OFC) of an organism. The present inventors performed a transcriptomics study on samples from the orbitofrontal cortex of 15 bipolar patients and 15 matched control post-mortem brains. The samples were analysed using Affymetrix HG-U133A Genechips, these are microarrays which comprise over 22,000 sequences. The OFC has not previously been investigated in microarray studies of bipolar disorder. A wide range of patient demographic variables were examined as potential confounding factors and were considered in subsequent statistical analyses. In the orbitofrontal cortex, 393 differentially expressed transcripts were identified by microarray analysis and a representative subset was validated by quantitative real-time PCR. Pathway analysis revealed significant up-regulation of genes involved in G-protein-coupled receptor signaling and response to stimulus (in particular the immune response), while genes relating to the ubiquitin cycle and intracellular transport showed coordinated down-regulation in bipolar disorder. Additionally several genes involved in synaptic function were significantly down-regulated in bipolar disorder.

These findings implicate the orbitofrontal cortex as a region prominently involved in affective disorders, such as bipolar disorder, depression and anxiety disorders, and the altered biological pathways indicate that diverse processes are affected. Overall, the results suggest that dysregulation of the ubiquitin pathway and synaptic function may be central to the disease process.

A method according to the invention may further comprise identification of side effect(s), if any, caused by administration of the candidate therapeutic agent.

The invention also provides a method of identifying a potential therapeutic agent for the prevention, treatment, or amelioration of an affective disorder, which comprises:
(a) providing a system capable of expressing one or more of the following genes: AVPR2, AZU1, CELSR1, DRD2, FZD2, GCG, GPR132, GPR4, HTR7, IL8RB, MS4A2, OPN1MW /// OPN1LW, OPRL1, OPRM1, OR3A2, OXTR, PARD3, RGS16, SSTR5, SSX2, VIPR2, FZD3, AZU1, C8A, CD7, CD72, CRIP1, GBX2, HLA-E, IL1R2, IL2RA, IL4, IL4R, IL5, IL8RB, KIR2DS1, KNG1, MS4A2, ORM1///ORM2, PFC, PRLR, TNFRSF17, SOCS5, WWP1, APG12L, FLJ11011, HIP2, PCGF4, PJA2, SENP6, TRIM23, UBE2G1, USP14, VPS41, WWP1, FBXL8, FLJ20315, BCAP29, C14orf108, COPA, FUSIP1, G3BP2, GRP58, HSPCA, IPO7, KIAA1012, KPNB1, RAB2, RANBP2, RBM8A, STAM, VPS41, STX11, TIMM17A, SYT1, CREB1, PREPL, SLC29A1 or MYT1;
(b) assessing the expression level of the gene(s) in the presence and absence of a candidate compound, and,
(c) identifying the candidate compound as a potential therapeutic agent if expression is modulated in the presence of the candidate compound such that:
   (i) one or more of the following genes is upregulated: FZD3, SOCS5, WWP1, APG12L, FLJ11011, HIP2, PCGF4, PJA2, SENP6, TRIM23, UBE2G1, USP14, VPS41, WWP1, BCAP29, C14orf108, COPA, FUSIP1, G3BP2, GRP58, HSPCA, IPO7, KIAA1012, KPNB1, RAB2, RANBP2, RBM8A, STAM, VPS41, SYT1, CREB1, PREPL and/or SLC29A1; and/or,
   (ii) one or more of the following genes is downregulated: AVPR2, AZU1, CELSR1, DRD2, FZD2, GCG, GPR132, GPR4, HTR7, IL8RB, MS4A2, OPN1MW /// OPN1LW, OPRL1, OPRM1, OR3A2, OXTR, PARD3, RGS16, SSTR5, SSX2, VIPR2, AZU1, C8A, CD7, CD72, CRIP1, GBX2, HLA-E, IL1R2, IL2RA, IL4, IL4R, IL5, IL8RB, KIR2DS1, KNG1, MS4A2, ORM1///ORM2, PFC, PRLR, TNFRSF17, FBXL8, FLJ20315; STX11, TIMM17A and/or MYT1.

The system may be an *in vitro* system capable of transcription of the gene and/or translation of mRNA encoding the protein coded by the gene. A preferred system is a cell, such as a cell derived from a human patient, or animal or animal model, or a cultured cell or cell line. Preferably, the cell is a neural cell, glial cell or other brain cell or cell line, or a peripheral cell (i.e. any non-central nervous system cell), such as a T-cell, B-cell, other blood cell, or fibroblasts; or a cell that has an expression profile that is typical of neural cell, glial cell or other brain cell or cell line, or a peripheral cell. Alternatively, the cell may be a cell that can be manipulated to produce an expression profile typical of neuronal cells, glial cell or other brain cell or cell line, or a peripheral cell.

Modulation of expression may be detected in systems such as cells by the effect on the phenotype of the cells, this may be detected, for example, after stimulating the cells, e.g. though receptor activation or blocking, for example using antibodies, growth factors, hormones or other signalling molecules. Where the gene or genes of interest are involved in the ubiquitin cycle, modulation of ubiquitin cycle genes may result in changes in protein aggregation, protein trafficking and protein localisation as well as affecting protein processing and folding.

The invention further provides a method of identifying a potential therapeutic agent for the prevention, treatment, or amelioration of an affective disorder which comprises:
(a) contacting one or more protein(s) encoded by a gene selected from: FZD3, SOCS5, WWP1, APG12L, FLJ11011, HIP2, PCGF4, PJA2, SENP6, TRIM23, UBE2G1, USP14, VPS41, WWP1, BCAP29, C14orf108, COPA, FUSIP1, G3BP2, GRP58, HSPCA, IPO7, KIAA1012, KPNB1, RAB2, RANBP2, RBM8A, STAM, VPS41, SYT1, CREB1, PREPL, SLC29A1; AVPR2, AZU1, CELSR1, DRD2, FZD2, GCG, GPR132, GPR4, HTR7, IL8RB, MS4A2, OPN1MW /// OPN1LW, OPRL1, OPRM1, OR3A2, OXTR, PARD3, RGS16, SSTR5, SSX2, VIPR2, AZU1, C8A, CD7, CD72, CRIP1, GBX2, HLA-E, IL1R2, IL2RA, IL4, IL4R, IL5, IL8RB, KIR2DS1, KNG1, MS4A2, ORM1///ORM2, PFC, PRLR, TNFRSF17, FBXL8, FLJ20315; STX11, TIMM17A and MYT1, and,
(b) determining the activity of the protein(s) in the presence and absence of the candidate compound, wherein the candidate compound is identified as a potential therapeutic agent if the activity of one or more of the proteins encoded by the following genes is enhanced: FZD3, SOCS5, WWP1, APG12L, FLJ11011, HIP2, PCGF4, PJA2, SENP6, TRIM23, UBE2G1, USP14, VPS41, WWP1, BCAP29, C14orf108, COPA, FUSIP1, G3BP2, GRP58, HSPCA, IPO7, KIAA1012, KPNB1, RAB2, RANBP2, RBM8A, STAM, VPS41, SYT1, CREB1, PREPL and/or SLC29A1; and/or; the activity of one or more of the proteins encoded by the following genes is inhibited: AVPR2, AZU1, CELSR1, DRD2, FZD2, GCG, GPR132, GPR4, HTR7, IL8RB, MS4A2, OPN1MW /// OPN1LW, OPRL1, OPRM1, OR3A2, OXTR, PARD3, RGS16, SSTR5, SSX2, VIPR2, AZU1, C8A, CD7, CD72, CRIP1, GBX2, HLA-E, IL1R2, IL2RA, IL4, IL4R, IL5, IL8RB, KIR2DS1, KNG1, MS4A2, ORM1///ORM2, PFC, PRLR, TNFRSF17, FBXL8, FLJ20315; STX11, TIMM17A and/or MYT1.

Methods suitable for assessing the activity of a protein are known in the art.
Assessment of activity can be made by detecting catalytic or enzymatic activity of the protein on a suitable substrate, detecting the induction of a reporter gene (e.g., a regulatory element that is responsive to the protein and which is operably linked to a nucleic acid encoding a detectable marker, e. g. luciferase), or detecting a cellular response associated with the gene product, such as induction of G-protein-coupled receptor signaling, or a downstream event associated with such signaling.

The candidate compound can be identified as a regulator of the activity of the protein and thus as a potential therapeutic agent, by comparing the activity of the protein in the presence and absence of the candidate compound.

The invention yet further provides a method of identifying a potential therapeutic agent for the prevention, treatment, or amelioration of an affective disorder which comprises:
(a) contacting one or more protein(s) encoded by a gene selected from: FZD3, SOCS5, WWP1, APG12L, FLJ11011, HIP2, PCGF4, PJA2, SENP6, TRIM23, UBE2G1, USP14, VPS41, WWP1, BCAP29, C14orf108, COPA, FUSIP1, G3BP2, GRP58, HSPCA, IPO7, KIAA1012, KPNB1, RAB2, RANBP2, RBM8A, STAM, VPS41, SYT1, CREB1, PREPL, SLC29A1; AVPR2, AZU1, CELSR1, DRD2, FZD2, GCG, GPR132, GPR4, HTR7, IL8RB, MS4A2, OPN1MW /// OPN1LW, OPRL1, OPRM1, OR3A2, OXTR, PARD3, RGS16, SSTR5, SSX2, VIPR2, AZU1, C8A, CD7, CD72, CRIP1, GBX2, HLA-E, IL1R2, IL2RA, IL4, IL4R, IL5, IL8RB, KIR2DS1, KNG1, MS4A2, ORM1///ORM2, PFC, PRLR, TNFRSF17, FBXL8, FLJ20315; STX11, TIMM17A and MYT1 with a binding partner of the protein in the absence and presence of a candidate compound, and
(b) assessing binding of the protein to its binding partner in the absence and presence of the candidate compound,
wherein the candidate regulator is identified as a potential therapeutic agent if the interaction of one or more of the following protein(s) with its binding partner is enhanced: FZD3, SOCS5, WWP1, APG12L, FLJ11011, HIP2, PCGF4, PJA2, SENP6, TRIM23, UBE2G1, USP14, VPS41, WWP1, BCAP29, C14orf108, COPA, FUSIP1, G3BP2, GRP58, HSPCA, IPO7, KIAA1012, KPNB1, RAB2, RANBP2, RBM8A, STAM, VPS41, SYT1, CREB1, PREPL and/or SLC29A1; and/or, the interaction of one or more of the following protein(s) with its binding partner is inhibited: AVPR2, AZU1, CELSR1, DRD2, FZD2, GCG, GPR132, GPR4, HTR7, IL8RB, MS4A2, OPN1MW /// OPN1LW, OPRL1, OPRM1, OR3A2, OXTR, PARD3, RGS16, SSTR5, SSX2, VIPR2, AZU1, C8A, CD7, CD72, CRIP1, GBX2, HLA-E, IL1R2, IL2RA, IL4, IL4R, IL5, IL8RB, KIR2DS1, KNG1, MS4A2, ORM1///ORM2, PFC, PRLR, TNFRSF17, FBXL8, FLJ20315; STX11, TIMM17A and/or MYT1.

In some methods described herein, a binding partner of an expression product of the gene may be used to detect the level of that expression product.

A binding partner may be a protein, and preferably is an antibody. The term "antibody" as used herein includes, but is not limited to: polyclonal, monoclonal, bispecific, humanised or chimeric antibodies, single chain antibodies, Fab fragments and F (ab')₂ fragments, fragments produced by a Fab expression library, anti-idiotypic (anti-Id) antibodies, and epitope-binding fragments of any of the above. The term "antibody" as used herein also refers to immunoglobulin molecules and immunologically-active portions of immunoglobulin molecules, i. e., molecules that contain an antigen binding site that specifically binds an antigen. The immunoglobulin molecules of the invention can be of any class (e. g., IgG, IgE, IgM, IgD and IgA) or subclass of immunoglobulin molecule.

The binding partner may be an aptamer, or oligonucleotide, or any synthetic chemical receptor or compound capable of specifically binding an expression product of the gene (which may be an mRNA or protein). The binding partner may comprise a detectable label, such as a luminescent, fluorescent or radioactive label and/or an affinity tag.

Additionally the invention provides a method of identifying a candidate binding partner as a therapeutic agent for the prevention, treatment, or amelioration of an affective disorder which comprises contacting one or more protein(s) encoded by a gene selected from: AVPR2, AZU1, CELSR1, DRD2, FZD2, GCG, GPR132, GPR4, HTR7, IL8RB, MS4A2, OPN1MW /// OPN1LW, OPRL1, OPRM1, OR3A2, OXTR, PARD3, RGS16, SSTR5, SSX2, VIPR2, FZD3, AZU1, C8A, CD7, CD72, CRIP1, GBX2, HLA-E, IL1R2, IL2RA, IL4, IL4R, IL5, IL8RB, KIR2DS1, KNG1, MS4A2, ORM1///ORM2, PFC, PRLR, TNFRSF17, SOCS5, WWP1, APG12L, FLJ11011, HIP2, PCGF4, PJA2, SENP6, TRIM23, UBE2G1, USP14, VPS41, WWP1, FBXL8, FLJ20315, BCAP29, C14orf108, COPA, FUSIP1, G3BP2, GRP58, HSPCA, IPO7, KIAA1012, KPNB1, RAB2, RANBP2, RBM8A, STAM, VPS41, STX11, TIMM17A, SYT1, CREB1, PREPL, SLC29A1 and MYT1; with a sample comprising a candidate binding partner, and assessing if the candidate binding partner binds to the protein.

Furthermore, the invention provides for the use of one or more of the following protein(s) or nucleic acid(s) in a method of identifying a potential therapeutic agent for the prevention, treatment, or amelioration of an affective disorder:
i) a protein encoded by a gene or a fragment thereof selected from: AVPR2, AZU1, CELSR1, DRD2, FZD2, GCG, GPR132, GPR4, HTR7, IL8RB, MS4A2, OPN1MW /// OPN1LW, OPRL1, OPRM1, OR3A2, OXTR, PARD3, RGS16, SSTR5, SSX2, VIPR2, FZD3, AZU1, C8A, CD7, CD72, CRIP1, GBX2, HLA-E, IL1R2, IL2RA, IL4, IL4R, IL5, IL8RB, KIR2DS1, KNG1, MS4A2, ORM1///ORM2, PFC, PRLR, TNFRSF17, SOCS5, WWP1, APG12L, FLJ11011, HIP2, PCGF4, PJA2, SENP6, TRIM23, UBE2G1, USP14, VPS41, WWP1, FBXL8, FLJ20315, BCAP29, C14orf108, COPA, FUSIP1, G3BP2, GRP58, HSPCA, IPO7, KIAA1012, KPNB1, RAB2, RANBP2, RBM8A, STAM, VPS41, STX11, TIMM17A, SYT1, CREB1, PREPL, SLC29A1 and MYT1; or,
ii) a nucleic acid encoding a protein of (i) above, or a fragment of said nucleic acid; and/or;
iii) a nucleic acid capable of hybridising to a nucleic acid according to (ii).

According to this aspect, the nucleic acid can be a single or double-stranded nucleic acid and can be DNA, RNA or a DNA/RNA duplex. The nucleic acid should hybridize specifically (for example under conditions of high stringency) to the nucleic acid expression product, or nucleic acid complementary to, or derived therefrom, so that the level of the nucleic acid expression product in the biological sample can be determined. A suitable nucleic acid binding partner is an oligonucleotide primer, such as is used for the synthesis of cDNA by reverse transcription from mRNA of the gene. In some preferred embodiments, the level of a nucleic acid expression product of the gene is determined by amplification of that nucleic acid expression product, for example by PCR. Thus, primers capable of amplifying the nucleic acid expression product are provided by the invention.

The invention also provides RNAi molecules, that is double stranded RNA molecules, comprising a nucleic acid sequence complementary to an mRNA sequence transcribed from a gene as described herein. RNAi molecules are typically in the range of from about 10 to about 40 nucleotides in length, suitably in the range of from about 20 to about 30 nucleotides in length. RNAi molecules may be used to regulate expression of a protein encoded by a gene as described herein. Thus, RNAi molecules may be employed as therapeutic compounds for reducing expression of one or more genes overexpressed in affective disorders as described herein. Suitably such RNAi may be administered intrathecally. The invention further provides pharmaceutical compositions comprising one or more RNAi and a pharmaceutically acceptable carrier, preferably formulated for intrathecal administration.

Additionally, RNAi molecules may be used to down-regulate, i.e. knockdown, expression of a gene as described herein, e.g. in a genetically modified animal or cell according to the invention.

Also within the scope of the present invention is the use of a regulator of expression of a protein encoded by a gene selected from: AVPR2, AZU1, CELSR1, DRD2, FZD2, GCG, GPR132, GPR4, HTR7, IL8RB, MS4A2, OPN1MW /// OPN1LW, OPRL1, OPRM1, OR3A2, OXTR, PARD3, RGS16, SSTR5, SSX2, VIPR2, FZD3, AZU1, C8A, CD7, CD72, CRIP1, GBX2, HLA-E, IL1R2, IL2RA, IL4, IL4R, IL5, IL8RB, KIR2DS1, KNG1, MS4A2, ORM1///ORM2, PFC, PRLR, TNFRSF17, SOCS5, WWP1, APG12L, FLJ11011, HIP2, PCGF4, PJA2, SENP6, TRIM23, UBE2G1, USP14, VPS41, WWP1, FBXL8, FLJ20315, BCAP29, C14orf108, COPA, FUSIP1, G3BP2, GRP58, HSPCA, IPO7, KIAA1012, KPNB1, RAB2, RANBP2, RBM8A, STAM, VPS41, STX11, TIMM17A, SYT1, CREB1, PREPL, SLC29A1 and MYT1, in a method of identifying a potential therapeutic agent for the prevention, treatment, or amelioration of an affective disorder.

The invention also provides the use of a binding partner of:
(i) a protein encoded by a gene or a fragment thereof selected from: AVPR2, AZU1, CELSR1, DRD2, FZD2, GCG, GPR132, GPR4, HTR7, IL8RB, MS4A2, OPN1MW /// OPN1LW, OPRL1, OPRM1, OR3A2, OXTR, PARD3, RGS16, SSTR5, SSX2, VIPR2, FZD3, AZU1, C8A, CD7, CD72, CRIP1, GBX2, HLA-E, IL1R2, IL2RA, IL4, IL4R, IL5, IL8RB, KIR2DS1, KNG1, MS4A2, ORM1///ORM2, PFC, PRLR, TNFRSF17, SOCS5, WWP1, APG12L, FLJ11011, HIP2, PCGF4, PJA2, SENP6, TRIM23, UBE2G1, USP14, VPS41, WWP1, FBXL8, FLJ20315, BCAP29, C14orf108, COPA, FUSIP1, G3BP2, GRP58, HSPCA, IPO7, KIAA1012, KPNB1, RAB2, RANBP2, RBM8A, STAM, VPS41, STX11, TIMM17A, SYT1, CREB1, PREPL, SLC29A1 and MYT1; or,
ii) a nucleic acid encoding a protein of (i) above, or a fragment of said nucleic acid; and/or;
iii) a nucleic acid capable of hybridising to a nucleic acid according to (ii).

In a method of identifying a potential therapeutic agent for the prevention, treatment, or amelioration of an affective disorder.

Furthermore, the invention provides the use of an expression vector comprising a nucleic acid according to the invention in a method of identifying a potential therapeutic agent for the prevention, treatment, or amelioration of an affective disorder.

Additionally, the invention provides the use of a cell or cell line capable of expressing a nucleic acid according to the invention in a method of identifying a potential therapeutic agent for the prevention, treatment, or amelioration of an affective disorder.

Preferably the cell(s) is a neural cell, glial cell, or other brain cell or peripheral cell or a cell line derived therefrom.

In preferred embodiments of the invention, screening for potential therapeutic agents is carried out by contacting a candidate therapeutic agent with cultured cells or cell lines.

Preferably, the cells are neural cells, glial cell or other brain cell or cell line, or are cells that have an expression profile that is typical of these types of cells or, alternatively, they may be cells that can be manipulated to produce an expression profile typical of these types of cells. The cell(s) may be genetically modified as described herein. The cells or cell lines used will also, preferably, give rise to reproducible changes in their gene expression profiles when contacted with one or more known antipsychiatric drugs (for example, valproate, Haloperidol, Pirenzepine, Perazine, Risperdal, Famotidine, Zyprexa, Clozaril, Mesoridazine, Quetiapine, Risperidone, Olanzapine, or Clozapine). In particularly preferred embodiments, these changes will be opposite changes to those that are observed in affective disorders, in particular in bipolar disorder. That is to say, in such embodiments, genes (or their homologs) normally expressed at higher levels in affective disorders, such as bipolar disorder, are preferably expressed at lower levels in cells or cell lines contacted with the known antipsychiatric drug, and vice ersa.

Alternatively, cells or cell lines may also be obtained from patients having an affective disorder, particularly a bipolar disorder, or from an animal model of an affective disorder, such as bipolar disorder; e.g. from a genetically modified non-human animal as described herein.

It will be appreciated that cells or cell lines used in the methods of this invention should be carefully stored, grown and manipulated to ensure that the phenotype of the cells and cell lines is maintained.

The invention further provides a genetically-modified non-human mammal or other non-human vertebrate or invertebrate organism, in which the level of expression of one or more gene(s) selected from AVPR2, AZU1, CELSR1, DRD2, FZD2, GCG, GPR132, GPR4, HTR7, IL8RB, MS4A2, OPN1MW /// OPN1LW, OPRL1, OPRM1, OR3A2, OXTR, PARD3, RGS16, SSTR5, SSX2, VIPR2, FZD3, AZU1, C8A, CD7, CD72, CRIP1, GBX2, HLA-E, IL1R2, IL2RA, IL4, IL4R, IL5, IL8RB, KIR2DS1, KNG1, MS4A2, ORM1///ORM2, PFC, PRLR, TNFRSF17, SOCS5, WWP1, APG12L, FLJ11011, HIP2, PCGF4, PJA2, SENP6, TRIM23, UBE2G1, USP14, VPS41, WWP1, FBXL8, FLJ20315, BCAP29, C14orf108, COPA, FUSIP1, G3BP2, GRP58, HSPCA, IPO7, KIAA1012, KPNB1, RAB2, RANBP2, RBM8A, STAM, VPS41, STX11, TIMM17A, SYT1, CREB1, PREPL, SLC29A1, MYT1; is altered compared to the level of expression in a corresponding wild type non-human mammal. In some embodiments of this aspect of the invention the expression of two or more of the genes is altered.

Expression of the gene or genes in the genetically modified animal may be increased or decreased, depending on the gene of interest. The genetic modification may be such that one or more of the gene(s) is knocked down, e.g. using RNAi, or "knocked-out", by deletion or other inactivation. One or more of the gene(s) may be upregulated, e.g. by providing a stronger promoter, or may be "knocked-in". Methods for providing animals with such genetic manipulations are well known to those of skill in the art.

A genetically modified non-human mammal is suitably selected from the group consisting of: a non-human primate, horse, cow, pig, sheep, dog, cat, rabbit, rodent; guinea pig, rat or mouse.

Preferably expression of one or more gene(s) selected from: FZD3, SOCS5, WWP1, APG12L, FLJ11011, HIP2, PCGF4, PJA2, SENP6, TRIM23, UBE2G1, USP14, VPS41, WWP1, BCAP29, C14orf108, COPA, FUSIP1, G3BP2, GRP58, HSPCA, IPO7, KIAA1012, KPNB1, RAB2, RANBP2, RBM8A, STAM, VPS41, SYT1, CREB1, PREPL and SLC29A1 is decreased in the genetically-modified recombinant animal.

Preferably expression of one or more gene(s) selected from: AVPR2, AZU1, CELSR1, DRD2, FZD2, GCG, GPR132, GPR4, HTR7, IL8RB, MS4A2, OPN1MW /// OPN1LW, OPRL1, OPRM1, OR3A2, OXTR, PARD3, RGS16, SSTR5, SSX2, VIPR2, AZU1, C8A, CD7, CD72, CRIP1, GBX2, HLA-E, IL1R2, IL2RA, IL4, IL4R, IL5, IL8RB, KIR2DS1, KNG1, MS4A2, ORM1///ORM2, PFC, PRLR, TNFRSF17, FBXL8, FLJ20315; STX11, TIMM17A and MYT1 is increased in the genetically-modified animal.

The invention also provides for the use of a genetically modified non-human mammal or other vertebrate or invertebrate organism according to the invention as an animal model for an affective disorder, preferably for bipolar disorder.

In other preferred embodiments of the invention, screening for potential therapeutic agents is carried out by administering a candidate therapeutic agent to an organism, preferably a non human animal (such as a mouse, rat, rabbit, non-human primate, e.g. monkey, guinea pig, dog or cat), although in some circumstances it may be desirable to administer a candidate therapeutic agent to a human, for example as part of a clinical trial.

It will be appreciated that where non-human cells, organisms, or extracts are used in accordance with the invention, the proteins and genes specified will normally refer to the appropriate homologues of the human proteins or genes (i.e. the equivalent proteins and genes in that non-human cell or organism) that are identified herein as being abnormally expressed in the orbitofrontal cortex of human bipolar disorder patients. In some embodiments (for example in some embodiments of the screening assays and methods described herein), the human gene (s) or protein (s) may be expressed in a non-human organism, cell, system, or extract.

The invention also provides a genetically modified cell, in which the level of expression of one or more gene selected from AVPR2, AZU1, CELSR1, DRD2, FZD2, GCG, GPR132, GPR4, HTR7, IL8RB, MS4A2, OPN1MW /// OPN1LW, OPRL1, OPRM1, OR3A2, OXTR, PARD3, RGS16, SSTR5, SSX2, VIPR2, FZD3, AZU1, C8A, CD7, CD72, CRIP1, GBX2, HLA-E, IL1R2, IL2RA, IL4, IL4R, IL5, IL8RB, KIR2DS1, KNG1, MS4A2, ORM1///ORM2, PFC, PRLR, TNFRSF17, SOCS5, WWP1, APG12L, FLJ11011, HIP2, PCGF4, PJA2, SENP6, TRIM23, UBE2G1, USP14, VPS41, WWP1, FBXL8, FLJ20315, BCAP29, C14orf108, COPA, FUSIP1, G3BP2, GRP58, HSPCA, IPO7, KIAA1012, KPNB1, RAB2, RANBP2, RBM8A, STAM, VPS41, STX11, TIMM17A, SYT1, CREB1, PREPL, SLC29A1 and MYT1; is altered compared to the level in a corresponding wild type cell. In some embodiments of this aspect of the invention expression of two or more of the genes is altered.

Yet further provided is the use of a genetically modified non-human mammal according to the invention, or a cell or cells obtained or derived therefrom, or a cell according to the invention, in a method of identifying a potential therapeutic agent for the prevention, treatment, or amelioration of an affective disorder.

The invention also provides for the use in a method of diagnosing whether a subject has, or is at risk of developing an affective disorder of:
(i) a binding partner of a nucleic acid encoding any of the genes selected from AVPR2, AZU1, CELSR1, DRD2, FZD2, GCG, GPR132, GPR4, HTR7, IL8RB, MS4A2, OPN1MW /// OPN1LW, OPRL1, OPRM1, OR3A2, OXTR, PARD3, RGS16, SSTR5, SSX2, VIPR2, FZD3, AZU1, C8A, CD7, CD72, CRIP1, GBX2, HLA-E, IL1R2, IL2RA, IL4, IL4R, IL5, IL8RB, KIR2DS1, KNG1, MS4A2, ORM1///ORM2, PFC, PRLR, TNFRSF17, SOCS5, WWP1, APG12L, FLJ11011, HIP2, PCGF4, PJA2, SENP6, TRIM23, UBE2G1, USP14, VPS41, WWP1, FBXL8, FLJ20315, BCAP29, C14orf108, COPA, FUSIP1, G3BP2, GRP58, HSPCA, IPO7, KIAA1012, KPNB1, RAB2, RANBP2, RBM8A, STAM, VPS41, STX11, TIMM17A, SYT1, CREB1, PREPL, SLC29A1 and MYT1; to determine the level of expression of the gene, or,
(ii) a binding partner of a protein encoded by a gene selected from AVPR2, AZU1, CELSR1, DRD2, FZD2, GCG, GPR132, GPR4, HTR7, IL8RB, MS4A2, OPN1MW /// OPN1LW, OPRL1, OPRM1, OR3A2, OXTR, PARD3, RGS16, SSTR5, SSX2, VIPR2, FZD3, AZU1, C8A, CD7, CD72, CRIP1, GBX2, HLA-E, IL1R2, IL2RA, IL4, IL4R, IL5, IL8RB, KIR2DS1, KNG1, MS4A2, ORM1///ORM2, PFC, PRLR, TNFRSF17, SOCS5, WWP1, APG12L, FLJ11011, HIP2, PCGF4, PJA2, SENP6, TRIM23, UBE2G1, USP14, VPS41, WWP1, FBXL8, FLJ20315, BCAP29, C14orf108, COPA, FUSIP1, G3BP2, GRP58, HSPCA, IPO7, KIAA1012, KPNB1, RAB2, RANBP2, RBM8A, STAM, VPS41, STX11, TIMM17A, SYT1, CREB1, PREPL, SLC29A1 and MYT1; to determine the level of expression of the gene.

The invention provides a method of diagnosing whether a subject has, or is at risk of developing an affective disorder, which comprises assessing the level of expression of one or more of gene(s) selected from: AVPR2, AZU1, CELSR1, DRD2, FZD2, GCG, GPR132, GPR4, HTR7, IL8RB, MS4A2, OPN1MW /// OPN1LW, OPRL1, OPRM1, OR3A2, OXTR, PARD3, RGS16, SSTR5, SSX2, VIPR2, FZD3, AZU1, C8A, CD7, CD72, CRIP1, GBX2, HLA-E, IL1R2, IL2RA, IL4, IL4R, IL5, IL8RB, KIR2DS1, KNG1, MS4A2, ORM1///ORM2, PFC, PRLR, TNFRSF17, SOCS5, WWP1, APG12L, FLJ11011, HIP2, PCGF4, PJA2, SENP6, TRIM23, UBE2G1, USP14, VPS41, WWP1, FBXL8, FLJ20315, BCAP29, C14orf108, COPA, FUSIP1, G3BP2, GRP58, HSPCA, IPO7, KIAA1012, KPNB1, RAB2, RANBP2, RBM8A, STAM, VPS41, STX11, TIMM17A, SYT1, CREB1, PREPL, SLC29A1 and MYT1; in a biological sample taken from the subject.

Also provided is a method of monitoring response to therapeutic intervention in a subject predisposed to or suffering from an affective disorder which comprises assessing the level of expression of one or more of gene(s) selected from: AVPR2, AZU1, CELSR1, DRD2, FZD2, GCG, GPR132, GPR4, HTR7, IL8RB, MS4A2, OPN1MW /// OPN1LW, OPRL1, OPRM1, OR3A2, OXTR, PARD3, RGS16, SSTR5, SSX2, VIPR2, FZD3, AZU1, C8A, CD7, CD72, CRIP1, GBX2, HLA-E, IL1R2, IL2RA, IL4, IL4R, IL5, IL8RB, KIR2DS1, KNG1, MS4A2, ORM1///ORM2, PFC, PRLR, TNFRSF17, SOCS5, WWP1, APG12L, FLJ11011, HIP2, PCGF4, PJA2, SENP6, TRIM23, UBE2G1, USP14, VPS41, WWP1, FBXL8, FLJ20315, BCAP29, C14orf108, COPA, FUSIP1, G3BP2, GRP58, HSPCA, IPO7, KIAA1012, KPNB1, RAB2, RANBP2, RBM8A, STAM, VPS41, STX11, TIMM17A, SYT1, CREB1, PREPL, SLC29A1 and MYT1; in a biological sample taken from the subject.

The level of expression can be assessed by assessing the level of mRNA of the one or more gene(s) present in a biological sample taken from a subject. Alternatively or additionally, the level of expression can be assessed by assessing the level of one or more protein(s) present in a biological sample taken from a subject, said protein being encoded by the one or more gene(s).

Suitably, the biological sample comprises a tissue or cell in which the level of expression correlates with level of expression of the gene in the orbitofrontal cortex.

The biological sample can be selected from the group consisting of: blood, serum, plasma, CSF, urine and tear fluid.

In methods of diagnosis, if the level of expression of the gene or genes is modulated as shown in Table 2 (and described in the summary of changes in gene expression identified by gene expression profiling), then a subject can be identified as having, or being at risk of developing, an affective disorder, in particular bipolar disorder. In methods of monitoring response to therapeutic intervention, a stabilisation or amelioration of the disorder can be identified if the level of expression of the gene or genes of interest is modulated in the opposite manner to the changes described in Table 2 (and described in the summary of changes in gene expression identified by gene expression profiling), i.e. if the level of gene expression is modulated towards the expression level in a normal subject.

Methods of the invention can be performed in array format, e.g. on a chip, or as a multiwell array. Methods can be adapted into platforms, systems or kits for single tests, or multiple identical or multiple non-identical tests, and can be performed in high throughput format. Methods of the invention may comprise performing one or more additional, different tests to confirm or exclude a result.

The invention also provides a method of prevention, treatment, or amelioration of an affective disorder which comprises increasing the level or activity of one or more of the following proteins in the brain, in particular in the orbitofrontal cortex, of a subject in need of such prevention, treatment, or amelioration: FZD3, SOCS5, WWP1, APG12L, FLJ11011, HIP2, PCGF4, PJA2, SENP6, TRIM23, UBE2G1, USP14, VPS41, WWP1, BCAP29, C14orf108, COPA, FUSIP1, G3BP2, GRP58, HSPCA, IPO7, KIAA1012, KPNB1, RAB2, RANBP2, RBM8A, STAM, VPS41, SYT1, CREB1, PREPL and/or SLC29A1.

The invention also provides a method of prevention, treatment, or amelioration of an affective disorder which comprises reducing the level or activity of one or more of the following proteins in the brain, in particular in the orbitofrontal cortex, of a subject in need of such prevention, treatment, or amelioration: AVPR2, AZU1, CELSR1, DRD2, FZD2, GCG, GPR132, GPR4, HTR7, IL8RB, MS4A2, OPN1MW /// OPN1LW, OPAL1, OPRM1, OR3A2, OXTR, PARD3, RGS16, SSTR5, SSX2, VIPR2, AZU1, C8A, CD7, CD72, CRIP1, GBX2, HLA-E, IL1R2, IL2RA, IL4, IL4R, IL5, IL8RB, KIR2DS1, KNG1, MS4A2, ORM1///ORM2, PFC, PRLR, TNFRSF17, FBXL8, FLJ20315; STX11, TIMM17A and/or MYT1.

Also provided by the invention is a solid support, e.g. in the form of an array, comprising a substrate to which is attached one or more probes capable of hybridising specifically to an mRNA transcribed from one or more genes selected only from the group consisting of: AVPR2, AZU1, CELSR1, DRD2, FZD2, GCG, GPR132, GPR4, HTR7, IL8RB, MS4A2, OPN1MW /// OPN1LW, OPRL1, OPRM1, OR3A2, OXTR, PARD3, RGS16, SSTR5, SSX2, VIPR2, FZD3, AZU1, C8A, CD7, CD72, CRIP1, GBX2, HLA-E, IL1R2, IL2RA, IL4, IL4R, IL5, IL8RB, KIR2DS1, KNG1, MS4A2, ORM1///ORM2, PFC, PRLR, TNFRSF17, SOCS5, WWP1, APG12L, FLJ11011, HIP2, PCGF4, PJA2, SENP6, TRIM23, UBE2G1, USP14, VPS41, WWP1, FBXL8, FLJ20315, BCAP29, C14orf108, COPA, FUSIP1, G3BP2, GRP58, HSPCA, IPO7, KIAA1012, KPNB1, RAB2, RANBP2, RBM8A, STAM, VPS41, STX11, TIMM17A, SYT1, CREB1, PREPL, SLC29A1 and MYT1.

In such arrays, identical probes can be immobilised at a plurality of loci on a solid substrate, or a different probe can be immobilised at each of a plurality of loci on a solid substrate. Suitably a probe is a nucleic acid capable of hybridising specifically to an mRNA of interest.

The invention also provides a solid support, such as a protein array, comprising one or more protein(s) or fragment(s) thereof encoded by one or more of the following genes: AVPR2, AZU1, CELSR1, DRD2, FZD2, GCG, GPR132, GPR4, HTR7, IL8RB, MS4A2, OPN1MW /// OPN1LW, OPRL1, OPRM1, OR3A2, OXTR, PARD3, RGS16, SSTR5, SSX2, VIPR2, FZD3, AZU1, C8A, CD7, CD72, CRIP1, GBX2, HLA-E, IL1R2, IL2RA, IL4, IL4R, IL5, IL8RB, KIR2DS1, KNG1, MS4A2, ORM1///ORM2, PFC, PRLR, TNFRSF17, SOCS5, WWP1, APG12L, FLJ11011, HIP2, PCGF4, PJA2, SENP6, TRIM23, UBE2G1, USP14, VPS41, WWP1, FBXL8, FLJ20315, BCAP29, C14orf108, COPA, FUSIP1, G3BP2, GRP58, HSPCA, IPO7, KIAA1012, KPNB1, RAB2, RANBP2, RBM8A, STAM, VPS41, STX11, TIMM17A, SYT1, CREB1, PREPL, SLC29A1 and/or MYT1.

In a protein array according to the invention, identical proteins can be immobilised at a plurality of loci on a solid substrate, or a different proteins can be immobilised at each of a plurality of loci on a solid substrate. The term protein refers to both full-length proteins and also to fragments thereof, e.g. domains or other protein fragments, suitably in the range of from about 10 to about 80 amino acids in length.

Also provided the invention is a solid support comprising one or more binding partner(s) of one or more protein(s), such as a binding partner array comprising one or more binding partner(s) of one or more protein(s), encoded by one or more of the following genes: AVPR2, AZU1, CELSR1, DRD2, FZD2, GCG, GPR132, GPR4, HTR7, IL8RB, MS4A2, OPN1MW /// OPN1LW, OPRL1, OPRM1, OR3A2, OXTR, PARD3, RGS16, SSTR5, SSX2, VIPR2, FZD3, AZU1, C8A, CD7, CD72, CRIP1, GBX2, HLA-E, IL1R2, IL2RA, IL4, IL4R, IL5, IL8RB, KIR2DS1, KNG1, MS4A2, ORMI///ORM2, PFC, PRLR, TNFRSF17, SOCS5, WWP1, APG12L, FLJ11011, HIP2, PCGF4, PJA2, SENP6, TRIM23, UBE2G1, USP14, VPS41, WWP1, FBXL8, FLJ20315, BCAP29, C14orf108, COPA, FUSIP1, G3BP2, GRP58, HSPCA, IPO7, KIAA1012, KPNB1, RAB2, RANBP2, RBM8A, STAM, VPS41, STX11, TIMM17A, SYT1, CREB1, PREPL, SLC29A1, and/or MYT1.

Suitably binding partners for inclusion in arrays include antibodies.

The term "antibody" as used herein includes, but is not limited to: polyclonal, monoclonal, bispecific, humanised or chimeric antibodies, single chain antibodies, Fab fragments and F (ab')₂ fragments, fragments produced by a Fab expression library, anti-idiotypic (anti-Id) antibodies, and epitope-binding fragments of any of the above. The term "antibody" as used herein also refers to immunoglobulin molecules and immunologically-active portions of immunoglobulin molecules, i. e., molecules that contain an antigen binding site that specifically binds an antigen. The immunoglobulin molecules of the invention can be of any class (e. g., IgG, IgE, IgM, IgD and IgA) or subclass of immunoglobulin molecule.

A further aspect of the invention provides novel binding partners, i.e. ligands, such as naturally occurring or chemically synthesised compounds, capable of specific binding to a protein or peptide encoded by a gene as described herein. A binding partner according to the invention may comprise a peptide, an antibody or a fragment thereof, or an aptamer or oligonucleotide, capable of specific binding to the protein or peptide. The antibody can be a monoclonal antibody or a fragment thereof capable of specific binding to the protein or peptide. A binding partner according to the invention may be labeled with a detectable marker, such as a luminescent, fluorescent or radioactive marker; alternatively or additionally, a binding partner according to the invention may comprise an affinity tag.

An support, such as an array, according to the invention can be used in a method for identifying a potential therapeutic agent for the prevention, treatment, or amelioration of an affective disorder, or in a method of diagnosis of an affective disorder or for monitoring an affective disorder, or for monitoring efficacy of therapeutic intervention in an affective disorder, or for selecting candidates for clinical trials.

Such methods may involve providing solid support, e.g. in the form of an array, having immobilised thereon protein encoded by the one or more gene(s) described herein or a fragment thereof, probing the immobilised protein or fragment thereof with its binding partner in the presence of a candidate therapeutic agent and detecting modulation of binding; wherein the candidate therapeutic agent is identified as a potential therapeutic agent if it is capable of modulating, i.e., inhibiting or enhancing, binding between the immobilised protein and the probe binding partner. In an alternative configuration, the binding partner is arrayed and the probe is a protein encoded by a gene described herein, or a fragment thereof, the binding partner and probe being capable of association in the absence of an inhibitor.

Suitably the probe is labelled with a detectable marker and binding of the probe is assessed by detection of the detectable marker. In preferred embodiments the detectable marker is a fluorescent, luminescent or radioactive marker. The detectable marker can be a particulate label such as an up-converting phosphor and/or quantum dots.

Binding of the probe can be assessed by a direct or indirect immunological method or by surface plasmon resonance.

Alternatively, the probe or immobilised moiety may be detected directly or indirectly via interaction with a ligand or ligands such as an antibody or a biomarker-binding fragment thereof, or other peptide, or ligand, e.g. aptamer, or oligonucleotide, capable of specifically binding the probe or immobilised moiety. The ligand may possess a detectable label, such as a luminescent, fluorescent or radioactive label, and/or an affinity tag.

The invention also provides a kit or other system (high-thoughput or otherwise) for use in a method for identifying a potential therapeutic agent for the prevention, treatment, or amelioration of an affective disorder or in a method of diagnosis or monitoring an affective disorder, the kit or system comprising means for detecting expression products, or nucleic acids derived from nucleic acid expression products, of one or more of the genes specified herein.

Such kits or or systems may comprise an array according to the invention.

A kit other system according to the invention, may comprise:
i) instructions for use of the kit; and/or,
ii) predetermined amount of an isolated expression product of one or more of the genes specified in claim 1 for use as a standard, or control.

The invention additionally provides a method for selecting a participant in a clinical trial to determine the effectiveness of a potential therapeutic agent for the prevention, treatment, or amelioration of an affective disorder, comprising: determining the level of expression of one or more of the gene(s) specified in herein in a biological sample obtained from the candidate participant; and selecting the candidate as a participant for the clinical trial if the level of expression of the gene or genes is altered compared to the level in a normal subject.

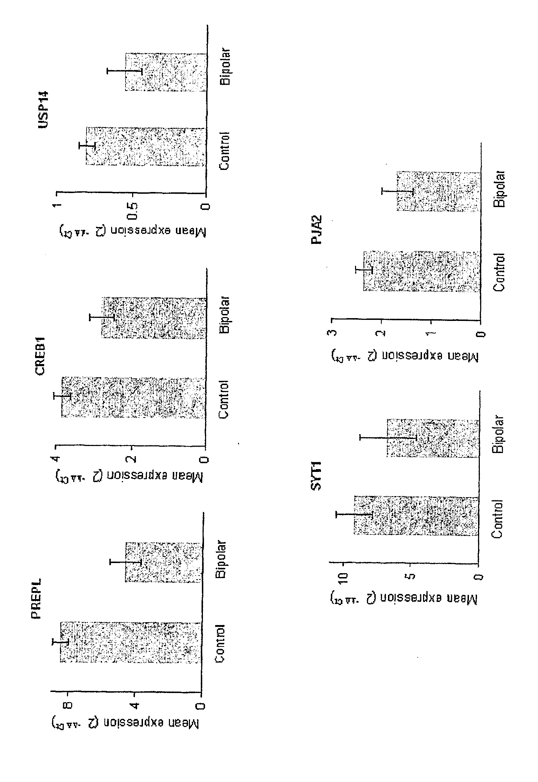

### Brief Description of the Drawing

The accompanying drawing shows graphical plots of quantitative real-time PCR results for validated genes.

The following Examples illustrate the invention.

### Tissue collection and RNA extraction

All procedures have previously been described¹³. In brief, fresh-frozen orbitofrontal cortex tissue from 15 bipolar and 15 well-matched control individuals were obtained from the Neuropathology Consortium of the Stanley brain collection (Stanley Medical Research Institute, US). Key demographic characteristics are summarized in Table 1.

Total RNA was extracted from post-mortem orbitofrontal cortices (Brodmann area 11) from bipolar patients and matched controls using Tri-reagent (Sigma, UK) or Trizol (Gibco-Brl). RNA quality was assessed using a high resolution electrophoresis system (Agilent Technologies, Palo Alto, CA, USA).

### Microarray analysis

Isolated total RNA was carried through the Affymetrix preparation protocol¹⁴, hybridized to HGU133A GeneChips (Affymetrix CA, USA) and subjected to stringent sample and data quality control steps as previously described¹³.

### Preprocessing of microarray data

Affymetrix Microarray Suite 5 (MAS5) was used for image processing and data acquisition. Rigorous quality control procedures^{13,15} resulted in the exclusion (blind to diagnosis) of nine OFC samples. Following elimination of these outlier chips, expression measures were computed using the robust multi-chip average (RMA) method¹⁶. Finally, the gene expression matrix was filtered to exclude control probesets and to include only probe-sets that had a 'class A' assignment, *i*.*e.* at least nine of its eleven probes perfectly match the target transcript sequence. This filtering step reduced the gene expression matrix to 19537 probe-sets.

### Detection of differentially expressed genes

In order to identify transcripts which are truly differentially regulated in bipolar disorder, it was important to control for the effects of confounding clinical and demographic variables. The most serious confounding variable in microarray studies of postmortem brain samples is tissue pH, as its effect on the global expression profile can be greater in magnitude than that of the factor of primary interest *i*.*e.* disease status¹⁷⁻¹⁸. The effects of medication were also considered. Data on exposure to lithium and sodium valproate were not available for all patients and so fluphenazine equivalent (measured in mg and log₁₀ transformed) was used as a measure of drug treatment. Analysis of covariance (ANCOVA) was used to identify differentially expressed genes whilst controlling for the effects of confounding variables. This involved fitting a linear model for the expression of each probe-set in order to partition the variability due to the disorder from the variability attributable to confounders, biological noise and experimental noise. Since the data are unbalanced (*i*.*e*. the number of replications is not constant for each treatment combination) a type III sums of squares ANCOVA was applied using the ssType3 function in S-Plus 6 (Insightful Corporation, Seattle, WA, USA). P-values were adjusted using the Benjamini-Hochberg false discovery rate (FDR) controlling procedure¹⁹ as implemented in the Bioconductor²⁰ package 'multtest'²¹.

Samples were analyzed and for the OFC samples no difference in mean tissue pH was found between the patient and control groups (Table 1). Therefore, for the OFC samples the ANCOVA model fitted for each gene included a main effect for patient group and fluphenazine equivalent only.

### Functional profiling of differentially expressed genes

Functional profiling of the lists of up and down-regulated genes was performed using GoSurfer²²⁻²³ and Onto-Express²⁴⁻²⁵. GoSurfer associates a user input list of Affymetrix probe-set identifiers with gene ontology (GO) terms and the results are visualized as a hierarchical tree. In addition to a list of probeset identifiers for the differentially regulated genes, GoSurfer also requires a gene information table in which the probe-set identifiers are annotated with gene name, Unigene ID, Locuslink ID and GO terms.

This gene information table was generated from data held by the NetAffx database²⁶ (version dated 7^{th} December 2004) and the GO database²⁷ (version dated 22nd March 2005) using Chiplnfo²⁸. GoSurfer was used to identify GO categories specifically up-regulated or down-regulated in BD by submitting the list of up-regulated genes and list of down-regulated transcripts simultaneously.

Onto-Express was run separately for up-regulated and down-regulated genes. A list of gene symbols was submitted as the input file and the reference file was a list of gene symbols for all 'Class A' probesets. The hyper-geometric distribution and FOR options were selected before running the program. Results were inspected in the tree and synchronised views²⁹, where custom abstraction levels can be selected for direct comparison to the GoSurfer output.

The list of differentially expressed genes was inspected manually to confirm the output of the functional profiling tools and to identify other genes that may be relevant to bipolar disorder.

### QPCR Validation

For real-time quantitative polymerase chain reaction (QPCR), complementary DNA was synthesised from 3 µg of total RNA with an oligonucleotide deoxythymidine primer and Superscript First-Strand synthesis system (Invitrogen). Eight TaqMan® gene expression assays (detailed in Table 3) were selected for validation from the significant microarray gene list and gene ontology categories. Three endogenous controls, with similar microarray expression levels to the target genes and consistency across all samples, were chosen. Endogenous control assay QPCR results were checked to ensure there was no significant difference between the cycle threshold (Ct) values for the disease group and the control group. The transferrin receptor (TFRC) gene was found to be the most suitable control gene and used for the normalization of all target genes in the OFC brain region. Standard curves were constructed for each assay to ensure amplification efficiencies were close to 100% and comparable across all assays. QPCR was performed using the Applied Biosystems 1700 Expression Array System following manufacturer's instructions. The comparative Ct method was employed for quantification of the transcript expression levels³⁰.Triplicate delta Ct values were generated for six assays and in duplicate for two; the median value in each case was used for subsequent analysis. Samples were removed from analysis if the standard deviation of replicates was greater than 1; 2-^{ΔΔC}T values were then calculated and box-plots constructed for each sample group. Consistent outliers were investigated and four samples were removed from the OFC dataset; two with poor cDNA profiles and two which failed our in-house microarray QC procedures. The relative expression was determined using Student's t-test. A one-tailed test was performed on the OFC samples as a directional change was expected.

### Results

Fifteen post-mortem brain samples from bipolar patients and fifteen healthy controls were investigated using microarray technology.

In the OFC, 393 transcripts were classified as differentially expressed (p<0.05); 238 transcripts being upregulated in bipolar disorder and 155 down-regulated. Gene expression changes in post-mortem brain tissue are usually modest (less than 2-fold) in studies of psychiatric disorders^{9, 31}; in this study 1 up- and 43 down-regulated transcripts changed by more than 1.5 fold between disease and control groups.

Three transcripts, synaptotagmin I (SYT1); ATPase, H+ transporting, lysosomal 70kDa, V1 subunit A (ATP6V1A) and paternally expressed 3 (PEG3), were decreased by more than 2-fold.

### Pathway Analysis

Pathway analysis using GoSurfer revealed 4 major GO categories (biological process branch) significantly altered in the OFC of bipolar patients (p<0.01). Two categories, i.e. G-protein coupled receptor (GPCR) signaling and response to stimulus (in particular immune response), were significantly upregulated while two others, i.e. intracellular transport and the ubiquitin cycle, were down-regulated. These pathways were also identified as the most over-represented in the differentially expressed transcript list using Onto-Express. The GPCR signaling pathway was significantly up-regulated (corrected p=0.003) as was response to stimulus (corrected p=0.033) while immune response showed a trend to being significantly up-regulated (corrected p=0.062). The number of genes identified in all three categories corresponds to the number of up-regulated genes listed in each category by GoSurfer. However, the total number of genes annotated with each GO term is not given by either program and would affect the calculation of significance. Intracellular transport was reported down-regulated (corrected p=0.012); Onto-Express annotated 11 genes in this category compared to 15 by GoSurfer. Similarly, ubiquitin cycle showed a trend towards being down-regulated (corrected p=0.066) but only 7 genes were included in this category compared to the 11 annotated by GoSurfer. GoSurfer gene ontology annotations were confirmed by other sources³². Hence it is likely that the corrected p-values reported by Onto-Express are underestimated for intracellular transport and ubiquitin cycle pathways. Thus, we are confident that the pathways noted here are significantly altered in BD.

Within the GPCR signaling pathway, twenty-one transcripts were up-regulated in BD; 15 of these encoded receptors including the dopamine receptor D2 and serotonin receptor 7 (Table 2). Transcripts for the opioid receptor, mu 1 (OPRM1) and the opiate receptor-like 1 (OPRL1) were increased in the bipolar group. Both genes are involved in regulating pain transmission and are co-expressed in certain neuronal populations³³. Other up-regulated transcripts of note include receptors for somatostatin (SSTR5), arginine vasopressin (AVPR2), oxytocin (OXTR) and vasoactive intestinal peptide (VIPR2).

Twenty transcripts involved in immune response were up-regulated in patients with bipolar disorder (Table 2). These include 6 genes and receptors of the interleukin family, together with SOCS5, a regulatory protein associated with inflammatory processes and involved in suppressing cytokine signaling. Up-regulation of azurocidin 1 (AZU1), a neutrophil-derived lysosome granule protein, could indicate increased numbers of circulating neutrophils. Other up-regulated transcripts associated with the immune response include several complement cascade components (ORM2, PFC and C8A) and TNFRSF17, a tumor necrosis factor receptor family member associated with B cell survival and autoimmunity.

Fifteen transcripts for genes involved in intracellular transport were decreased in the brains of subjects with bipolar disorder (Table 2). Seven of these relate to the more specific process of nucleocytoplasmic transport. Nuclear import of proteins, vital for correct cellular targeting, is mediated by the karyopherin (importin) family of transport molecules³⁴. Karyopherin (importin) beta 1 (KPNB1) and importin 7 (IPO7) were down-regulated at the transcript level. A highly significant down-regulation of IPO7 was detected by two independent probe-sets (p=0.00036 and p=0.018) and the gene is located at 11p15, a region implicated in BD by a number of linkage and gene association studies35 (OMIM 125480). Additionally, the transcript for RAN binding protein 2 (RANBP2) showed a 1.58 fold reduction in the BD group and codes for a scaffolding protein where import/export complexes are assembled and disassembled.

Thirteen transcripts relating to the ubiquitin cycle were down-regulated in bipolar disorder (Table 2). Three of these represent genes of the ubiquitin-conjugating enzyme family (E2): ubiquitin-conjugating enzyme E2G 1 (UBE2G1); huntingtin interacting protein 2 (HIP2) and a hypothetical protein (FLJ11011). HIP2 may play a role in both Huntington's³⁶ and Alzheimer's disease³⁷. Six transcripts decreased in BD have ubiquitin protein ligase activity (WWP1, TRIM23, PJA2, PCGF4, HIP2 and VPS41); the ligases (E3) are the key regulatory determinants in ubiquitination³⁸. Another gene downregulated in BD was ubiquitin-specific protease 14 (USP14), which encodes a deubiquitinating enzyme and maps to chromosome 18p11, a well-established susceptibility locus for both BD and schizophrenia³⁹. Although not annotated with the GO term 'ubiquitin cycle', RAD23 homolog B (*S. cerevisiae*) contains an N-terminal ubiquitin-like domain (Ubl) which interacts with the 26S proteasome⁴⁰. The transcript for RAD23B was down-regulated 1.4 fold in BD and the protein product is thought to shuttle ubiquitinated substrates to the proteasome⁴¹.

Other dysregulated transcripts of interest included synaptotagmin I (SYT1), a synaptic vesicle protein. SYT1 showed a 2.44 fold decrease in expression in bipolar disorder, the greatest fold change of all differentially expressed transcripts identified.

Prolyl endopeptidase-like (PREPL) was decreased 1.7 fold in bipolar patients and a transcript for prolyl endopeptidase (PREP) itself showed decreased expression in the disease group but did not reach significance. PREP is thought to be involved in the maturation and degradation of its neuropeptide substrates, including substance P, oxytocin, vasopressin and angiotensin⁴².

### QPCR Validation

Using GPCR, the significant down-regulation of five of eight genes examined was confirmed in the OFC. These genes were PJA2, USP14, CREB1, PREPL and SYT1. Furthermore, the fold changes obtained using data derived from microarray and QPCR investigations were all comparable (Table 2). The up-regulation of HTR7 could not be validated by QPCR; this was most likely due to the low levels of expression of this transcript in both disease and control samples.

QPCR showed two ubiquitin-related genes were significantly decreased in samples from the dorsolateral prefrontal cortex DLPFC.

In summary, increased expression of numerous cell surface receptors may affect the response to extracellular stimuli, including neurotransmitters. Downstream effects may include disturbances within second-messenger signaling cascades, eventually leading to altered transcription patterns. There is strong evidence for abnormalities in signal transduction in BD, especially an enhanced response which could correspond to the observed receptor up-regulation at the transcript level.

Prolyl endopeptidase-like (PREPL) also showed a large decrease in expression in bipolar disorder, determined as a -1.70-fold decrease by microarray and as a -1.83-fold decrease by QPCR in the OFC. Activity of prolyl endopeptidase (PREP) in human brain is highest in frontal, parietal and occipital cortices⁴³ and plasma concentrations of this enzyme are elevated in mania and decreased in depression⁴⁴. The role of PREP in the maturation/degradation of neuropeptide signaling molecules may be important, particularly as levels of some neuropeptides (substance P, somatostatin and vasopressin) are also reported to be altered in affective disorders^{4s,46}. Interestingly, the common action of three mainstream mood stabilising drugs (lithium, sodium valproate and carbamazepine) on neurons was abolished by inhibiting PREP activity⁴⁷.

### References

1. OMIM Record 125480. National Center for Biotechnology Information website. Available at: http://www.ncbi.nlm.nih.gov/entrez/dispomim.cgi?id=125480. Accessed 2005.
2. OMIM Record 309200. National Center for Biotechnology Information website. Available at: http://www.ncbi.nlm.nih.gov/entrez/dispomim.cgi?id=309200. Accessed 2005.
3. Prabakaran et al, Mol Psychiatry. 2004;9:684-697, 643.
4. Middleton et al, J Neurosci. 2002;22:2718-2729.
5. Vawter et al, Schizophr. Res. 2002;58:11-20.
6. Hakak et al, Proc Natl Acad Sci U S A. 2001;98:4746-4751.
7. Mimics et al, Neuron. 2000;28:53-67.
8. Iwamoto et al, Hum Mol Genet. 2005;14:241-253.
9. Iwamoto et al, Mol. Psychiatry. 2004;9:406-416.
10. Konradi et al, Arch Gen Psychiatry. 2004;61:300-308.
11. Tkachev et al, Lancet. 2003;362:798-805.
12. Bezchlibnyk et al, J Neurochem. 2001;79:826-834.
13. Ryan et al, Biol Psychiatry. 2004;55:329-336.
14. Affymetrix. Available at: www.affymetrix.com. Accessed 2005.
15. AffyPLM: Fitting probe level models. Bioconductor website. Available at: http://www.bioconductor.org/repository/devel/vignette/AffyExtensions.pdf. Accessed 2005.
16. Irizarry et al, Biostatistics. 2003;4:249-264.
17. Tomita et al, Biol Psychiatry. 2004;55:346-352.
18. Li JZ et al, Hum Mol Genet. 2004;13:609-616.
19. Benjamini and Hochberg, Journal of the Royal Statistical Society B. 1995;57:289-300.
20. Bioconductor, open source software for bioinformatics. Available at: http://www.bioconductor.org/. Accessed 2005.
21. Pollard *et al*, *UC Berkeley Division of Biostatistics Working Paper Series* [serial online]. December 2004; Working paper 164.
22. Zhong et al, Appl Bioinformatics. 2004;3:261-264.
23. GoSurfer. Available at: http://www.biostat.harvard.edu/complab/gosurfer/. Accessed
24. Khatri et al, Genomics. 2002;79:266-270.
25. Onto-Express. Available at: http://vortex.cs.wayne.edu/research.htm. Accessed 2005.
26. Liu et al, Nucleic Acids Res. 2003;31:82-86.
27. Gene Ontology Database. The Gene Ontology Project. Available at: http://www.geneontology.org/. Accessed 2005.
28. Zhong et al, Nucleic Acids Res. 2003;31:3483-3486.
29. Khatri et al, Nucleic Acids Res. 2004;32:W449-456.
30. Livak et al, Methods. 2001;25:402-408.
31. Bosetti et al, Brain Res Bull. 2005;65:331-338.
32. Entrez Gene; Entrez PubMed. National Center for Biotechnolgy Information website. Available at: http://www.ncbi.nlm.nih.gov/. Accessed 2005.
33. Wang et al, J. Neurochem. 2005;92:1285-1294.
34. Pemberton et al, Traffic. 2005;6:187-198.
35. Petronis et al, Am J Med Genet C. Semin Med Genet. 2003;123:65-75.
36. Tanno et al, J Chem Neuroanat. 1999;17:99-107.
37. Song et al, Trends Mol Med. 2004;10:565-570.
38. Pickart et al, Biochim Biophys Acta. 2004;1695:55-72.
39. Berrettini, Biol Psychiatry. 2000;47:245-251.
40. Fujiwara et al, J Biol Chem. 2004;279:4760-4767.
41. Johnston and Madura, Prog Neurobiol. 2004;73:227-257.
42. Cunningham et al, Biochim Biophys Acta. 1997;1343:160-186.
43. Irazusta et al, Neurochem Int. 2002;40:337-345.
44. Maes et al, Psychiatry Res. 1995;58:217-225.
45. Rubinow, Biol Psychiatry. 1986;21:341-365.
46. Holsboer, J Neural Transm Suppl. 2003:17-34.
47. Williams et al, Nature. 2002;417:292-295.
48. Ogden et al, Mol Psychiatry. 2004;9:1007-1029.

**Table 1. Summary of patient demographics**

| **Demographic Variable** | **Bipolar** | **Control** | **T-test / Fisher exact test p-value†** |
|---|---|---|---|
| *(a) Orbitofrontal cortex group* | | | |
| *N* | 10 | 11 | N/A |
| Age (years) | 43.5 ± 10.7* | 47.8 ± 11.0* | 0.374 |
| Gender (F/M) | 5/5 | 4/7 | 0.670 |
| Age onset (years) | 20.1 ± 7.5* | N/A | N/A |
| Duration of illness (years) | 22.4 ± 10.2* | N/A | N/A |
| Fluphenazine mg. Equivalents | 23540 ± 24331* | 0 ± 0* | **0.014** |
| PMI (hours) | 33.8 ± 18.5* | 23.1 ± 9.6* | 0.124 |
| Side of brain (UR) | 5/5 | 6/5 | 1.000 |
| Brain pH | 6.17 ± 0.25* | 6.31 ± 0.22* | 0.187 |

| | | | |
|---|---|---|---|
| * mean ± SD t *t*-test used for metric variables; Fisher's exact test used for nominal variables. | | | |

**Table 2. Details of Differentially Expressed Genes within the Major Biological Pathways Dysregulated in Bipolar Disorder**

| **Gene Title** | **Representative Public ID** | **Gene Symbol** | **Chromosomal Location** | **Fold Change** | **P-value** |
|---|---|---|---|---|---|
| **G-PROTEIN COUPLED RECEPTOR SIGNALLING PATHWAY** | | | | | |
| Arginine vasopressin receptor 2 (nephrogenic diabetes insipidus) | NM_000054 | AVPR2 | chrXq28* | **1.25** | 0.021 |
| Azurocidin 1 (cationic antimicrobial protein 37) | NM_001700 | AZU1 | chr19p13.3 | **1.25** | 0.020 |
| Cadherin, EGF LAG seven pass G-type receptor 1 (flamingo homolog, Drosophila) | BC000059 | CELSR1 | chr22q13.3* | **1.19** | 0.020 |
| Dopamine receptor D2 /// dopamine receptor D2 | M30625 | DRD2 | chr11q23* | **1.23** | 0.025 |
| Frizzled homolog 2 (Drosophila) | L37882 | FZD2 | chr17q21.1 | **1.19** | 0.021 |
| Glucagon | NM_002054 | GCG | chr2q36-q37 | **1.15** | 0.029 |
| G protein-coupled receptor 132 | NM_013345 | GPR132 | chr14q32.3* | **1.06** | 0.045 |
| G protein-coupled receptor 4 | NM_005282 | GPR4 | chr19q13.3 | **1.23** | 0.021 |
| 5-hydroxytryptamine (serotonin) receptor 7 (adenylate cyclase-coupled) | NM_019859 | HTR7 | chr10q21-q24 | **1.19** | 0.027 |
| Interleukin 8 receptor, beta | NM_001557 | IL8RB | chr2q35 | **1.12** | 0.027 |
| Membrane-spanning 4 domains, subfamily A, member 2 (Fc fragment of gE, high affinity I, receptor for; beta polypeptide) | D10583 | MS4A2 | chr11q13 | **1.12** | 0.037 |
| Opsin 1 (cone pigments), medium-wave-sensitive (color blindness, deutan) /// opsin 1 (cone pigments), long-wave- sensitive (color blindness, protan) | NM_000513 | OPN1MW /// OPN1LW | chrXq28* | **1.21** | 0.020 |
| Opiate receptor-like 1 | AF348323 | OPRL1 | chr20q13.33* | **1.16** | 0.044 |
| Opioid receptor, mu 1 | L29301 | OPRM1 | chr6q24-q25 | **1.08** | 0.040 |
| Olfactory receptor, family 3, subfamily A, member 2 | NM 002551 | OR3A2 | chr17p13.3 | **1.15** | 0.047 |
| Oxytocin receptor | NM_000916 | OXTR | chr3p25 | **1.24** | 0.046 |
| Par-3 partitioning defective 3 homolog (C. elegans) | NM_019619 | PARD3 | chr10p11.22-p11.21 | **1.27** | 0.039 |
| Regulator of G-protein signalling 16 | U94829 | RGS16 | chr1q25-q31 | **1.16** | 0.045 |
| Somatostatin receptor 5 | NM_001053 | SSTR5 | chr16p13.3* | **1.23** | 0.044 |
| Synovial sarcoma, X breakpoint 2 | NM_003147 | SSX2 | chrXp11.23-p11.22 | **1.21** | 0.026 |
| Vasoactive intestinal peptide receptor 2 | NM_003382 | VIPR2 | chr7q36.3 | **1.12** | 0.046 |
| Frizzled homolog 3 (Drosophila) | NM_017412 | FZD3 | chr8p21 | **-1.27** | 0.036 |

| **IMMUNE RESPONSE** | | | | | |
|---|---|---|---|---|---|
| Azurocidin 1 (cationic antimicrobial protein 37) | NM_001700 | AZU1 | chr19p13.3* | **1.25** | 0.020 |
| Complement component 8, alpha polypeptide | NM_000562 | C8A | chr1p32 | **1.17** | 0.045 |
| CD7 antigen (p41) | NM_006137 | CD7 | chr17q25.2-q25.3 | **1.13** | 0.047 |
| CD72 antigen | AF283777 | CD72 | chr9p13.3 | **1.19** | 0.029 |
| Cysteine-rich protein 1 (intestinal) | NM_001311 | CRIP1 * | chr14q32.33 | **1.23** | 0.042 |
| Gastrulation brain homeo box 2 | AF118452 | GBX2 | chr2q37 | **1.25** | 0.048 |
| Major histocompatibility complex, class I, E | X56841 | HLA-E | chr6p21.3 | **1.43** | 0.042 |
| Interleukin 1 receptor, type II | U64094 | IL1R2 | chr2q12-q22* | **1.15** | 0.036 |
| Interleukin 2 receptor, alpha | NM_000417 | IL2RA | chr10p15-p14 | **1.24** | 0.048 |
| Interleukin 4 | NM_000589 | IL4 | chr5q31.1* | **1.20** | 0.029 |
| Interleukin 4 receptor | NM_000418 | IL4R | chr16p11.2-12.1* | **1.31** | 0.021 |
| Interleukin 5 (colony stimulating factor, eosinophil) | NM_000879 | IL5 | chr5q31.1* | **1.15** | 0.032 |
| Interleukin 8 receptor, beta | NM_001557 | IL8RB | chr2q35 | **1.12** | 0.027 |
| Killer cell immunoglobulin-like receptor, two domains, short cytoplasmic tail, 1 | NM_014512 | KIR2DS1 | chr19q13.4 | **1.15** | 0.021 |
| Kininogen 1 | NM_000893 | KNG1 | chr3q27 | **1.22** | 0.043 |
| Membrane-spanning 4 domains, subfamily A, member 2 (Fc fragment of IgE, high affinity I, receptor for; beta polypeptide) | D10583 | MS4A2 | chr11q13 | **1.12** | 0.037 |
| Orosomucoid 1 /// orosomucoid | NM_000607 | ORM1/// ORM2 | chr9q31-q32/// chr9q32* | **1.04** | 0.029 |
| Properdin P factor, complement 2 | NM_002621 | PFC | chrXp11.3-p11.23 | **1.13** | 0.025 |
| Prolactin receptor /// prolactin receptor | AF349939 | PRLR | chr5p14-p13 | **1.13** | 0.040 |
| Tumor necrosis factor receptor superfamily, member 17 | NM_001192 | TNFRSF17 | chr16p13.1* | **1.12** | 0.029 |
| Suppressor of cytokine signaling 5 | AW664421 | SOCS5 | chr2p21* | **-1.36** | 0.017 |
| WW domain containing E3 ubiquitin protein ligase 1 | BF131791 | WWP1 | chr8q21 | **-1.57** | 0.005 |

| **UBIQUITIN CYCLE** | | | | | |
|---|---|---|---|---|---|
| APG12 autophagy 12-like (S. cerevisiae) | BE965998 | APG12L | chr5q21-q22 | **-1.37** | 0.030 |
| Hypothetical protein FLJ11011 | NM_018299 | FLJ11011 | chr8q21.11 | **-1.23** | 0.030 |
| Huntingtin interacting protein 2 | NM_005339 | HIP2 | chr4p14 | **-1.49** | 0.029 |
| Polycomb group ring finger 4 | NM_005180 | PCGF4 | chr10p11.23 | **-1.40** | 0.045 |
| Praja 2, RING-H2 motif containing | AA142966 | PJA2 | chr5q21.3 | **-1.52** | 0.017 |
| SUMO1/sentrin specific protease 6 | NM_015571 | SENP6 | chr6q13-q14.3 | **-1.11** | 0.049 |
| Tripartite motif-containing 23 | AI021991 | TRIM23 | chr5q12.3 | **-1.64** | 0.010 |
| Ubiquitin-conjugating enzyme E2G 1 (UBC7 homolog, C. elegans) | BC002775 | UBE2G1 | chr1q42\|17p13.2 | **-1.53** | 0.021 |
| Ubiquitin specific protease 14 (tRNA-guanine transglycosylase) | NM_005151 | USP14 | chr18p11.32* | **-1.36** | 0.044 |
| Vacuolar protein sorting 41 (yeast) | NM_014396 | VPS41 | chr7p14-p13 | **-1.12** | 0.045 |
| WW domain containing E3 ubiquitin protein ligase 1 | BF131791 | WWP1 | chr8q21 | **-1.57** | 0.005 |
| F-box and leucine-rich repeat protein 8 | NM_018378 | FBXL8 | chr16q22.1 | **1.18** | 0.030 |
| Hypothetical protein FLJ20315 | NM_017763 | FLJ20315 | chr17q23.2 | **1.20** | 0.018 |
| | | | | | |

| **INTRACELLULAR TRANSPORT** | | | | | |
|---|---|---|---|---|---|
| B-cell receptor-associated protein 29 | NM_018844 | BCAP29 | chr7q22-q31 | **-1.28** | 0.031 |
| Chromosome 14 open reading frame 108 | NM_018229 | C14orf108 | chr14q22.3* | **-1.34** | 0.047 |
| Coatomer protein complex, subunit alpha | U24105 | COPA | chr1q23-q25 | **-1.31** | 0.042 |
| FUS interacting protein (serine-arginine rich) 1 | AF047448 | FUSIP1 | chr1p36.11* | **-1.16** | 0.042 |
| Ras-GTPase activating protein SH3 domainbinding protein 2 | AU149503 | G3BP2 | chr4q21.1 | **-1.58** | 0.037 |
| Glucose regulated protein, 58kDa | D83485 | GRP58 | chr15q15* | **-1.24** | 0.050 |
| Heat shock 90kDa protein 1, alpha | BG420237 | HSPCA | chr14q32.33* | **-1.51** | 0.042 |
| ^{a}Importin 7 | AL137335 | IPO7 | chr11p15.4* | **-1.38** | 0.000 |
| KIAA1012 | NM_014939 | KIAA1012 | chr18q12.1* | **-1.27** | 0.045 |
| Karyopherin (importin) beta 1 | BC003572 | KPNB1 | chr17q21.32 | **-1.08** | 0.049 |
| ^{a}RAB2, member RAS oncogene family RAN binding protein 2 | AU158062 | RAB2 | chr8q12.1 | **-1.57** | 0.020 |
| | AI681120 | RANBP2 | ch r2q 12.3 | **-1.58** | 0.027 |
| RNA binding motif protein 8A Vacuolar protein sorting 41 (yeast) | AF182415 | RBM8A | chr1q12 | **-1.19** | 0.033 |
| | NM_003473 | STAM | chr10p14-p13 | **-1.42** | 0.046 |
| Syntaxin 11 | NM_014396 | VPS41 | chr7p14-p13 | **-1.12** | 0.045 |
| Translocase of inner mitochondrial membrane 17 homolog A (yeast) | AF071504 | STX11 | chr6q24.2 | **1.26** | 0.036 |
| **OTHER** | **NM_006335 I NM 006335** | **TIMM17A** | chr1q32.1* | **1.17** | 0.020 |
| Synaptotagmin I | | | | | |
| cAMP responsive element binding protein 1 | AV723167 | SYT1 | chr12cen-q21 | **-2.44** | 0.039 |
| Prolyl endopeptidase-like | AA161486 | CREB1 | chr2q34 | **-1.48** | 0.025 |
| Solute carrier family 29 (nucleoside transporters), member 1 | AW000954 | PREPL | chr2p22.1 | **-1.70** | 0.044 |
| ^{a}Myelin transcription factor 1 | AF079117 | SLC29A1 | chr6p21.1-p21.2 | **-1.32** | 0.045 |
| | AB020642 | MYT1 | chr20q13.33 | **1.18** | 0.013 |

| | | | | | |
|---|---|---|---|---|---|
| * susceptibility locus for bipolar disorder ^{a} two significant probe-sets for gene | | | | | |

**Table 3. Comparison of microarray and QPCR results for gene expression in the OFC and DLPFC of bipolar disorder**

| | | **OFC (n=24)** | | | |
|---|---|---|---|---|---|
| | | **Microarray** | | **QPCR** | |
| *Target Gene* | *Category* | *Fold Change¹* | *P-value²* | *Fold Change* | *P*-*value* |
| HIP2 | Ubiquitin | **-1.49** | **0.029** | -1.24 | 0.129 |
| PJA2 | Ubiquitin | **-1.52** | **0.017** | **-1.41** | **0.041** |
| USP14 | Ubiquitin | **-1.36** | **0.044** | **-1.45** | **0.035** |
| CREB1 | Synapse | **-1.48** | **0.025** | **-1.36** | **0.010** |
| PREPL | Synapse | **-1.70** | **0.044** | **-1.83** | **0.001** |
| SYT1 | Synapse | **-2.44** | **0.039** | **-1.77** | **0.024** |
| DRD2 | Receptor | **1.23** | **0.025** | **-1.60** | **0.009** |
| HTR7 | Receptor | **1.19** | **0.027** | **-1.08** | **0.407** |

| | | | | | |
|---|---|---|---|---|---|
| ¹ Fold changes were calculated by comparison of the mean expression value in the bipolar group to the control group. Negative values indicate a decrease in expression in bipolar disorder. ² P-values were calculated using 2-tailed Student's t-test for microarray data. A one-tailed t-test was used for QPCR validation in OFC as a directional change was expected. P<0.05 is indicated with bold font. Abbreviations: QPCR, quantitative real-time polymerase chain reaction; OFC, orbitofrontal cortex. | | | | | |

### Summary of Changes in Gene Expression identified by Gene Expression Profiling:

**G-PROTEIN COUPLED RECEPTOR SIGNALLING PATHWAY**
Increased
AVPR2, AZU1, CELSR1, DRD2, FZD2, GCG, GPR132, GPR4, HTR7, IL8RB, MS4A2, OPN1MW /// OPN1LW, OPRL1, OPRM1, OR3A2, OXTR, PARD3, RGS16, SSTR5,
SSX2, VIPR2,
Decreased
FZD3.
**IMMUNE RESPONSE**
Increased
AZU1, C8A, CD7, CD72, CRIP1 *, GBX2, HLA-E, IL1R2, IL2RA, IL4, IL4R, IL5, IL8RB,
KIR2DS1, KNG1, MS4A2, ORM1///ORM2, PFC, PRLR, TNFRSF17,
Decreased
SOCS5, WWP1
**UBIQUITIN CYCLE**
Decreased
APG12L, FLJ11011, HIP2, PCGF4, PJA2, SENP6, TRIM23, UBE2G1, USP14, VPS41,
WWP1,
Increased
FBXL8, FLJ20315
**INTRACELLULAR TRANSPORT**
Decreased
BCAP29, C14orf108, COPA, FUSIP1, G3BP2, GRP58, HSPCA, IPO7, KIAA1012,
KPNB1, RAB2, RANBP2, RBM8A, STAM, VPS41,
Increased
STX11, TIMM17A
**OTHER**
Decreased SYT1, CREB1, PREPL, SLC29A1, Increased MYT1

## Claims

1. A method of identifying a potential therapeutic agent for the prevention, treatment or amelioration of an affective disorder, which comprises:
(a) contacting a cell with a candidate therapeutic agent *in vitro*;
(b) determining whether expression of the gene PREPL is modulated in the cell in response to the candidate therapeutic agent; and
(c) identifying the candidate as a potential therapeutic agent if expression of the gene is increased.

2. A method according to claim 1, which comprises comparing the expression level of the gene in the presence and absence of the candidate therapeutic agent.

3. A method according to claim 1 or claim 2, wherein the cell is a neuronal cell, glial cell or other brain cell, or a peripheral cell.

4. A method of identifying a potential therapeutic agent for the prevention, treatment or amelioration of an affective disorder, which comprises:
(a) contacting one or more proteins encoded by the gene PREPL with a candidate compound; and
(b) determining the activity of the one or more proteins in the presence and absence of the candidate compound,
wherein the candidate compound is identified as a potential therapeutic agent if the activity of one or more of the proteins encoded by the gene is enhanced.

5. A method of diagnosing whether a subject has, or is at risk of developing, an affective disorder, which comprises assessing the level of expression of the gene PREPL in a biological sample taken from the subject.

6. A method of monitoring response to therapeutic intervention in a subject predisposed to or suffering from an affective disorder, which comprises assessing the level of expression of the gene PREPL in a biological sample, preferably selected from blood, serum, plasma, cerebrospinal fluid, urine and tear fluids taken from the subject.

7. A method according to claim 5 or claim 6, wherein the biological sample comprises a tissue or cell in which the level of expression correlates with level of expression of the gene in the orbitofrontal cortex.

8. A method according to any preceding claim, wherein the affective disorder is bipolar disorder.

9. Use of one or more of the following proteins or nucleic acids in a method of identifying a potential therapeutic agent for the prevention, treatment or amelioration of an affective disorder:
(i) a protein encoded by the gene PREPL or a fragment thereof;
(ii) a nucleic acid encoding a protein of (i) above, or a fragment of said nucleic acid; and/or
(iii) a nucleic acid capable of hybridising to a nucleic acid according to (ii).

10. Use of a binding partner of
(a) a protein encoded by the gene PREPL or fragment thereof; or
(b) a nucleic acid specified in claim 9 (ii) or (iii), in a method of identifying a potential therapeutic agent for the prevention, treatment or amelioration of an affective disorder.

11. A device comprising a substrate to which is attached an array of one or more probes capable of hybridising specifically to an mRNA transcribed from the gene PREPL.

12. Use of a device according to claim 11, in a method for identifying a potential therapeutic agent for the prevention, treatment or amelioration of an affective disorder, or in a method of diagnosis of an affective disorder, or for monitoring an affective disorder, or for monitoring efficacy of therapeutic intervention in an affective disorder, or for selecting candidates for clinical trials.

13. Use according to any of claims 9, 10 or 12, wherein the affective disorder is bipolar disorder.

14. A product which increases the level or activity in the brain, in particular in the orbitofrontal cortex, of the protein PREPL, for the prevention, treatment, or amelioration of an affective disorder.

15. A product according to claim 14, wherein the affective disorder is bipolar disorder.
